# FASCICULE DE BREVET EUROPEEN

(11) **EP 2 583 667 B1**
(45) Date de publication et mention de la délivrance du brevet: **16.01.2019**
(21) Numéro de dépôt: 11392009.4
(22) Date de dépôt: 20.10.2011
(51) Int. Cl.: A61K 8/37, A61K 8/92, A61Q 3/00, A61Q 7/00, A61P 17/14

(54) **Complexe lipidique pour favoriser la repousse des phanères et compositions associées**
Lipidkomplex zur Förderung des Nachwachsens von Hautanhangsgebilden, und entsprechende Zusammensetzungen
Lipid complex for promoting nail and hair growth and associated compositions

(43) Date de publication de la demande: 24.04.2013
(73) Titulaire: Les Laboratoires Asepta, 98000 Monaco (FR)
(72) Inventeur: Dencausse, Laurent, 06000 Nice (FR); Clamou, Jean-Luc, 06000 Nice (FR); Lacroix, Georges, 06000 Nice (FR); Laforet, Sonia, 06000 Nice (FR)
(74) Mandataire: Brizio Delaporte, Allison

(56) Documents cités:
- EP-A1- 1 072 251
- EP-A1- 2 345 401
- EP-A2- 0 839 515
- WO-A1-01/52873
- DE-A1- 4 129 066
- FR-A1- 2 774 585
- FR-A1- 2 856 067
- US-A1- 2010 048 598
- DATABASE GNPD [Online] Mintel; février 2006 (2006-02), Anonyme: "Beauty Elixer", XP002671682, Database accession no. 10248946
- DATABASE GNPD [Online] Mintel; février 2007 (2007-02), Anonyme: "In-Shower Moisturiser", XP002671683, Database accession no. 660521
- DATABASE GNPD [Online] Mintel; février 2008 (2008-02), Anonyme: "Nourishing Concentrate with Plant Omega 3, 6 and 9", XP002671684, Database accession no. 861433
- DATABASE GNPD [Online] Mintel; 7 février 2008 (2008-02-07), Anonyme: "Wrinkle Smoothing Eye Crème", XP002671685, Database accession no. 936491
- BONJEAN ALAIN ET AL: "Cameline - Camelina sativa (L.) Crantz: an opportunity for European agriculture and industry", O.C.L. OLEAGINEUX CORPS GRAS LIPIDES, EDITIONS JOHN LIBBEY EUROTEXT, MONTROUGE, FR, vol. 6, no. 1, 1 janvier 1999 (1999-01-01) , pages 28-34, XP009114039, ISSN: 1258-8210
- DATABASE GNPD [Online] MINTEL; September 2009 (2009-09), Anonyme: "El' Treament Serum", Database accession no. 1169742

## Description

La présente invention concerne un complexe lipidique et une composition cosmétique ou dermatologique à base dudit complexe pour la croissance et le renouvellement des phanères.

Le complexe et la composition associée sont destinés à être appliqués sur les phanères, incluant les cheveux, les cils et les ongles, pour favoriser leur pousse et/ou diminuer leur chute et à inhiber certains micro-organismes rencontrés dans la séborrhée du cuir chevelu. Le mode d'application du complexe et de la composition selon l'invention est une application dermique.

L'invention trouvera plus particulièrement son application pour des cas d'alopécies séborrhéiques ainsi que des alopécies diffuses aiguës d'origine réactionnelle dans un but principalement esthétique ou pour lutter contre des déficiences de l'organisme et des agressions externes.

L'alopécie désigne l'accélération de la chute des cheveux et/ou des poils. Les cheveux poussent selon un rythme cyclique et périodique variable selon les individus (âge, sexe...). Le cycle qui comporte trois phases se renouvelle en moyenne tous les trois ans chez l'homme et tous les cinq ans chez la femme. Les phases de croissance (phase anagène), de régression (phase catagène) et de repos (phase télogène) se succèdent jusqu'à la chute normale du cheveu. Ce cycle fait intervenir des phénomènes complexes où les facteurs génétiques et hormonaux jouent un rôle important. Un cheveu dont le cycle de vie s'accélère est la première caractéristique des alopécies androgénétiques. Le trouble vient de ce que les androgènes hâtent le rythme de la phase anagène, forçant le cheveu à passer trop vite en phase télogène et ne laissant pas au follicule pileux assez de temps pour fabriquer une kératine de qualité.

Dans 95 % des cas, l'hyperactivité d'une enzyme, la 5 alpha-réductase est la principale cause de cette pathologie (alopécie ou calvitie). En effet, la 5-alpha réductase transforme la testostérone (hormone naturellement présente chez l'homme et en quantité moins importante chez la femme) en dihydrotestostérone (DHT). Ces deux hormones sont véhiculées dans le sang et se fixent à une glycoprotéine (SHBG, sex hormon binding globulin) avant de migrer vers les follicules pileux. Un taux trop élevé de 5 alpha réductase provoque un raccourcissement de la phase anagène (phase de croissance) et une miniaturisation des cheveux. De ce fait, les cycles pilaires vont s'épuiser beaucoup plus rapidement et induire progressivement une calvitie.

Par ailleurs, ce phénomène s'accompagne généralement d'une hypertrophie de la glande sébacée avec pour conséquences des infiltrations séborrhéiques au niveau du follicule pileux. Bloqué sous le cuir chevelu, l'excès de sébum engorge les follicules pileux à la base, lieu où sont situées les cellules de reproduction du cheveu. Ce phénomène de séborrhée conduit à l'apparition de plaques et de bouchons favorisant le développement de microorganismes (bactéries et levures) et l'apparition de démangeaisons.

On connaît de nombreuses compositions formées à base d'huiles et de graisses animales pour lutter contre l'alopécie et améliorer les qualités de croissance des phanères. Ces compositions sont fabriquées à partir de graisses sous cutanées d'animaux (mustélidés, bovins, équidés) ou d'animaux marins (gadidés, squalidés).

Les résultats liés à l'utilisation de telles compositions sont très variables en fonction des individus, des procédés d'extraction et des animaux utilisés.

Les compositions proposées jusqu'à présent n'ont pas donné entière satisfaction.

Il existe donc le besoin de proposer une composition pour favoriser la pousse des phanères qui donne des résultats satisfaisants et réguliers.

A cet effet, la présente invention concerne un complexe lipidique à base d'huiles végétales. Le complexe comprend un mélange de trois huiles végétales dont l'huile de macadamia, l'huile de cameline et l'huile de pépins de raisin.

Le déposant a constaté que le complexe lipidique selon l'invention améliore de manière non contestable la croissance des phanères et que la fiabilité de la formulation du complexe lipidique selon l'invention était nettement supérieure à celle des compositions de l'état de la technique.

Le choix d'un complexe lipidique issu du végétal apporte également sécurité et confort pour les utilisateurs. En effet, il s'est avéré que les compositions produites à partir de graisses animales n'étaient pas toujours composées des mêmes composants. En fonction de l'animal, de son état de santé, de son régime alimentaire, la composition de la graisse extraite était différente, influençant grandement les effets du produit. En outre, l'utilisation de graisses et huiles animales peut être mal perçue par certaines personnes notamment en occident.

Selon l'invention, le complexe lipidique comprend également au moins un ester de palmitate et au moins un ester de myristate d'origine végétale.

Le complexe lipidique est avantageusement complété par au moins un dérivé synthétique, N-acylamino ester, obtenu par transformation chimique d'hydrolysats de protéines animales ou végétales préalablement enrichis en acides aminés soufrés. A titre d'exemple, il est synthétisé à partir d'hydrolysat de soie, de blé ou de soja.

Selon l'invention, le complexe lipidique peut être enrichi en vitamine E.

D'autres buts et avantages apparaîtront au cours de la description qui suit qui présente un mode de réalisation détaillé de l'invention qui n'a cependant pas pour but de la limiter.

Nous rappelons que l'invention concerne un complexe lipidique destiné à favoriser la croissance des phanères caractérisé par le fait qu'il comprend un mélange d'huile de macadamia, d'huile de cameline et d'huile de pépins de raisin, ainsi que deux esters, tel que défini dans la revendication 1.

Suivants des variantes préférées mais non limitatives, le complexe lipidique selon l'invention est tel que :
- les esters d'acide myristique et d'acide palmitique sont choisis parmi les esters méthyliques, éthyliques, propyliques ou isopropyliques.
- il comprend au moins un N-acylamino ester enrichi en au moins un acide aminé soufré.
- le au moins un N-acylamino ester est synthétisé à partir d'hydrolysat de soie.
- au moins un acide aminé soufré est choisi parmi la cystine, la cystéine, l'homocystéine, la méthionine et la taurine pris individuellement ou en mélange.
- le au moins un N-acylamino ester enrichi en acides aminés soufrés représente de 0,1 à 1 % en poids du complexe.
- il comprend de la vitamine E préférentiellement entre 0,1 et 0,5 % en poids du complexe.
- il comprend en poids du poids total du complexe :

| | |
|---|---|
| - Huile de macadamia | 44,0 % (± 20 %) ; |
| - Huile de cameline | 26,0 % (± 20 %) ; |
| - Huile de pépins de raisins | 10,0 % (± 10 %) ; |
| - Palmitate d'isopropyle | 17,0 % (± 10 %) ; |
| - Myristate d'isopropyle | 3,0 % (± 10 %) ; |
| - Vitamine E | 0,2 % (± 5 %). |

- il comprend en poids du poids total du complexe :

| | |
|---|---|
| - Huile de macadamia | 44,0 % ; |
| - Huile de cameline | 26,0 % ; |
| - Huile de pépins de raisins | 10,0 % ; |
| - Palmitate d'isopropyle | 17,0 % ; |
| - Myristate d'isopropyle | 2,7 % ; |
| - Vitamine E | 0,2 % ; |
| - N-acylamino ester de soie enrichi en acides aminés soufrés | 0,1 %. |

L'invention concerne également une composition comprenant le complexe lipidique qui représente avantageusement de 1 à 50% en poids de la composition.

Avantageusement la composition est sous forme de lotion capillaire, de masque capillaire, d'ampoule capillaire ou de shampoing capillaire ou de liquide ou de crème pour ongles et cils.

Le complexe lipidique selon l'invention comprend un mélange d'huile de macadamia, d'huile de cameline et d'huile de pépins de raisin. Le déposant s'est aperçu qu'un mélange des trois huiles citées ci-dessus donne des résultats très satisfaisants.

Le complexe lipidique selon l'invention est un mélange équilibré riche en acides gras mono-insaturés de 44 à 49 % et polyinsaturés de 20 à 25 % qui est particulièrement favorable pour la constitution des membranes cellulaires sous forme de phospholipides et d'esters de cholestérol. Par ailleurs, l'analyse de la fraction insaponifiable du mélange a démontré une quantité de tocophérols, tocotriénols et phytostérols importante qui contribue à une action antioxydante efficace sur les membranes cellulaires.

L'huile de macadamia est extraite des noix de macadamia, *Macadamia Ternifolia,* provenant d'un arbre tropical poussant en Australie, en Afrique orientale et dans les îles Hawaï. La noix contient une amande blanche très riche en huile, environ 76 %, et de qualité supérieure. L'huile de macadamia est choisie car le déposant a remarqué une bonne tolérance vis-à-vis notamment du cuir chevelu et une stabilité satisfaisante pour une application cosmétique et/ou dermatologique.

L'huile de macadamia renferme de façon tout à fait caractéristique 80 % d'acides gras mono-insaturés. Notamment l'acide palmitoléique (C16:1), de 20 à 30%, et l'acide oléique (C18:1 n-9) de 50 à 60%. De manière surprenante, la composition en acides gras de l'huile de macadamia est très proche du sébum humain. Or, contrairement à ce que l'homme du métier pourrait penser, un apport additionnel d'acides gras proches de la composition du sébum est un élément indispensable au gainage des fibres capillaires. En effet, lors d'alopécie, le sébum reste bloqué au niveau du bulbe pileux empêchant ainsi sa répartition sur la longueur des cheveux.

La fraction insaponifiable de l'huile, soit 0,3 %, massique d'huile traitée renferme des phytostérols, principalement du sitostérol, de 901 à 1354 microgrammes/g, du delta-5 avénastérol, de 82 à 207 microgrammes/g, du campestérol, de 61 à 112 microgrammes/g et du stigmastérol, de 8 à 19 microgrammes/g.

L'huile renferme peu de tocophérols mais est caractérisée par de forts taux de tocotriénol, de 31 à 92 microgrammes/g et de squalène, de 72 à 171 microgrammes/g.

L'huile de macadamia représente de 30 à 55% en poids du poids total du complexe lipidique, préférentiellement de l'ordre de 44% ± 20% de préférence 44%.

L'huile de cameline est extraite de la cameline, *Camelina Sativa,* qui est une plante de la famille des Crucifères (variété Glabrata).

Les graines de cameline renferment 40 % d'huile fortement polyinsaturée. La fraction d'acide gras polinsaturés est de 40 à 60 % en poids du poids total. Avec l'huile de lin et de l'huile de pourpier, l'huile de cameline est l'huile la plus riche en acide alpha-linolénique (C18:3 n-3) avec plus de 30%. Elle est donc très riche en oméga 3.

Cette huile se caractérise également par la présence d'acide gondoïque (C20:1 n-9) environ 15 %, d'acide érucique (C22:1n-9) environ 3% et par une proportion importante de gamma-tocophérols, de 651 à 922 microgrammes/g. Les principaux phytostérols sont le sitostérol, environ 300 microgrammes/g, le campestérol, environ 117 microgrammes/g, le delta-5 avénastérol, environ 37 microgrammes/g, et le brassicastérol caractéristique des crucifères environ 27 microgrammes/g.

L'huile de cameline représente de 20 à 35% en poids du poids total du complexe lipidique, préférentiellement de l'ordre de 26% ± 20% préférentiellement 26%.

L'huile de pépins de raisin, *Vitis Vinifera,* est originaire d'Asie mineure. La vigne croît spontanément dans les régions méridionales d'Europe, d'Afrique du Nord et au Moyen-Orient. Le raisin contient 2 à 4 pépins dont on extrait 12 à 13% d'huile.

L'huile de pépins de raisin apporte des polyphénols, environ de 59 à 115,5 microgrammes/g, et des tocophérols, environ 217 microgrammes/g, qui sont d'excellents agents anti-oxydants. L'huile de pépins de raisin est riche en acide linoléique (C18:2 n-6) ou oméga 6, de 58 à 78 %. La composition stérolique comprend le sitostérol majoritaire de 64 à 70 microgrammes/g, à côté duquel on trouve le campestérol de 7,5 à 14 microgrammes/g et de stigmastérol de 7,5 à 12 microgrammes/g.

L'huile de pépins de raisin représente de 9 à 11% en poids du poids total du complexe lipidique, préférentiellement de l'ordre de 10% ± 1% de préférence 10%.

**Tableau 1 - Compositions expérimentales en acides gras des composés du complexe lipidique selon l'invention**

| | **Myristate Isopropyle** | **Palmitate Isopropyle** | **Huile de pépins de raisins** | **Huile de macadamia** | **Huile de cameline** |
|---|---|---|---|---|---|
| **C14:0** | 98,99 | 0,54 | 0,05 | 0,77 | 0,06 |
| **C16:0** | 0,65 | 99,11 | 7,75 | 9 | 6,07 |
| **C16:1** | | 0,21 | 0,11 | 20,16 | 0,21 |
| **C18:0** | | 0,12 | 3,58 | 3,25 | 2,48 |
| **C18:1** | | 0,02 | 25,37 | 58,71 | 20,23 |
| **C18:2** | | | 61,92 | 3,88 | 20,93 |
| **C18:3** | | | 0,82 | 0,15 | 35,35 |
| **C20:0** | | | 0,23 | 1,98 | 1,36 |
| **C20:1** | 0,36 | | 0,17 | 1,9 | 13,31 |
| **autres** | | | 0 | 0,2 | 0 |
| **Total** | 100 | 100 | 100 | 100 | 100 |

Le demandeur a constaté de manière surprenante que le mélange de ces trois huiles dans le complexe lipidique de l'invention en plus d'apporter différents composés essentiels pour favoriser la pousse des phanères, permet de potentialiser leurs actions respectives notamment l'huile de macadamia qui améliore la qualité de la kératine des cheveux voit son effet augmenté par l'huile de cameline qui améliore l'hydratation de la peau et des phanères et favorise ainsi la pénétration des autres composés actifs.

Le complexe lipidique selon l'invention comprend des acides gras essentiels, acide linoléique (oméga 6) et alpha linolénique (oméga 3).

Au niveau du système capillaire, ils assurent ensemble la qualité des cheveux : les omégas 6 contrôlent la déperdition d'eau du cheveu et donc son hydratation, tandis que les omégas 3 maintiennent son élasticité.

Les acides gras essentiels s'incorporent dans les membranes des cellules dermiques qui entourent le follicule pileux. Leur rôle est de maintenir la peau du cuir chevelu à un taux d'hydratation correcte et de lui conférer élasticité et résistance. On a pu remarquer qu'un apport d'acides gras essentiels dans un complexe lipidique selon l'invention dans cette zone limite le dessèchement du cuir chevelu, les états pelliculaires. La kératine en formation est hydratée et plus résistante.

L'huile de macadamia facilite la micro-circulation et tonifie le système lymphatique. C'est une huile très pénétrante qui présente un toucher sec et ne crée pas de film occlusif. Ainsi, l'huile de macadamia est un agent de vectorisation idéal qui facilite la pénétration des huiles associées, cameline et pépins de raisin, et favorise le transfert trans-cutanée des acides gras essentiels et des antioxydants présents dans le complexe lipidique.

De plus, l'origine végétale du complexe assure une tolérance élevée.

Le complexe lipidique comprend également des esters d'acides végétaux: d'acide palmitique et d'acide myristique.

Les esters sont choisis parmi les esters méthyliques, éthyliques, propyliques ou isopropyliques. Le myristate et le palmitate d'isopropyle sont préférés. Les esters de palmitate et de myristate végétaux sont particulièrement intéressants dans le domaine de la cosmétique. Ils sont une source d'acide gras saturés en C14 et C16.

L'ester d'acide myristique d'origine végétale représente de 1 à 15% en poids du complexe et l'ester d'acide palmitique d'origine végétale représente de 5 à 30% en poids du complexe.

Préférentiellement, les esters de palmitate représentent de 15 à 20% en poids du poids total du complexe lipidique, plus préférentiellement de l'ordre de 17% ± 10% de préférence 17% et les esters de myristate représentent de 2 à 5% en poids du poids total du complexe lipidique, plus préférentiellement de l'ordre de 3% ± 10% de préférence 3%.

Les esters de palmitate et de myristate ont des propriétés émollientes et lubrifiantes non occlusives. Ils sont donc très intéressants dans le traitement de l'alopécie où ils assouplissent la peau du cuir chevelu en améliorant la pénétration des composés actifs sans boucher les follicules pileux.

Avantageusement, le complexe lipidique est enrichi en tocophérols, également dénommés vitamine E, qui sont des espèces liposolubles ayant une activité antioxydante qui freine le vieillissement cutané et assure la stabilité des structures cellulaires.

Par ailleurs, la vitamine E en s'opposant à la formation de radicaux libres permet de prévenir la formation de peroxydes lipidiques, de radicaux libres et de nitrosamines, au sein des produits cosmétiques, permettant ainsi de maintenir la stabilité des émulsions et de prolonger leur durée de conservation. Au niveau capillaire, il a pu être constaté que la vitamine E, en limitant la peroxydation des lipides du sébum, diminue les phénomènes d'irritation du cuir chevelu.

Préférentiellement, les tocophérols issus de la vitamine E représentent de 0,1 à 0,5% du poids du poids total du complexe lipidique, plus préférentiellement de l'ordre de 0,2% ± 5% de préférence 0,2%.

Selon un mode de réalisation avantageux, le complexe lipidique peut être complété massiquement par des N-acylamino esters enrichis en acides aminés soufrés. Ces composés sont également dénommés Thio-Lipesters. Ils sont préférentiellement issus d'hydrolysat de soie, de blé ou de soja. L'adition de thiolipesters dans le complexe lipidique selon l'invention a montré une forte potentialisation de la croissance des phanères.

Les N-acylamino esters enrichis en acides aminés soufrés sont des dérivés synthétiques obtenus à partir d'un hydrolysat de protéines par exemple protéines animales telle que la soie, protéines sécrétées par le *Bombyx Mori,* ou protéines végétales de blé ou de soja, préalablement enrichis en acides aminés ou dérivés exclusivement soufrés tels que la cystéine, l'homocystéine, la cystine, la méthionine et la taurine pris individuellement ou en mélange. L'hydrolysat de soie est préféré car il a pu être constaté que son utilisation dans le complexe lipidique améliore l'hydratation des phanères. De plus, les protéines de soie ont des affinités très fortes avec les kératines des cheveux, des ongles.

Les acides aminés soufrés sont choisis parmi ceux dont la configuration naturelle est de type L, tel que rencontré dans la nature de sorte à assurer une bonne tolérance lors de l'application du complexe lipidique.

Il est particulièrement avantageux de suppléer les N-acylamino esters en acides aminés soufrés qui améliorent l'effet de potentialisation de N- acylamino esters. Selon une possibilité, cette amélioration des résultats peut s'expliquer par le fait que les acides aminés soufrés sont présents dans les kératines et notamment dans les phanères en proportion importante de l'ordre de 14 % et donc leur présence semble essentielle dans la synthèse des kératines ce qui augmente la croissance des ongles, des cheveux et améliore la solidité des repousses. La L-cystéine et la L-méthionine sont préférées pour supplémenter l'hydrolysat de soie notamment en raison de leur prédominance dans la kératine des cheveux. Par ailleurs, la taurine qui est un dérivé d'acide aminé soufré sera choisie pour son action protectrice spécifique au niveau du bulbe capillaire en s'opposant au processus de déformation et de compression du bulbe.

Préférentiellement, les N-acylamino esters enrichis en acides aminés soufrés représentent de 0,05 à 5% en poids du poids total du complexe lipidique, plus préférentiellement de 0,01 à 2%, encore plus préférentiellement de l'ordre de 0,1%.

On a pu constater qu'une quantité supérieure à 5% de N-acylamino esters pouvait avoir un effet délétère pour les cellules vivantes des phanères. Il est préféré de rester sous un seuil de 1%. A titre préféré, une quantité de l'ordre de 0,1% de N-acylamino esters apporte une bonne efficacité de pousse des phanères sans effet néfaste.

Les N-acylamino esters enrichis en acides aminés soufrés ont également une activité bactéricide intéressante. En effet, l'alopécie s'accompagne généralement d'une hypertrophie de la glande sébacée avec pour conséquences des infiltrations séborrhéiques au niveau du follicule pileux. Ce phénomène de séborrhée conduit à l'apparition de plaques et de bouchons favorisant le développement de microorganismes (bactéries et levures) et l'apparition de démangeaisons. Les N-acylamino esters enrichis en acides aminés soufrés en plus d'améliorer la pousse des phanères contribuent à réduire le développement des micro-organismes permettant au follicule pileux à nouveau de fabriquer des cellules de cheveux.

La composition massique en acides aminés de la protéine de soie qui est utilisée comme substrat protéique est donnée dans le tableau 2.

**Tableau 2**

| Aminogramme des acides aminés atomisés de la soie (% m/m) | | | |
|---|---|---|---|
| Acides aminés | % massiques | Acides aminés | % massiques |
| Glycine | 45,3 | Tyrosine | 0,1 |
| Alanine | 42,9 | Isoleucine | traces |
| Sérine | 10,3 | Méthionine | traces |
| A. Glutamique | 0,5 | Phénylalanine | traces |
| Valine | 0,5 | Leucine | traces |
| A. Aspartique | 0,4 | Cystéine | traces |

Le pourcentage initial d'acides aminés soufrés dans la soie utilisée est quasi nul. L'addition de cystine, d'homocystéine, de méthionine, de cystéine ou de taurine pris individuellement ou en mélange au substrat protéique permet d'augmenter artificiellement les proportions globales d'acides aminés soufrés.

### Exemple 1 -

### Préparation des N-acvlamino esters de soie enrichi L-méthionine.

### 1/ Préparation du mélange hydrolysat de soie + L-méthionine

Dans deux récipients indépendants peser séparément 4,25 Kg d'Hydrolysat de soie et 0,75 Kg de L-méthionine. Ces quantités correspondent aux proportions massiques 85/15. Une fois les pesées réalisées réunir les 5,0 kg dans le réacteur.

### 2/ Estérification du mélange d'acides aminés

Dans un réacteur de capacité d'environ 200 litres, le mélange d'acides aminés (55,75 moles) est chargé puis additionné de 70 litres de méthanol puis dans l'ampoule de coulée de 8 kg de SOCI2 (soit 1,2 eq/AA, 67 moles ou 4,9 litres). Le chlorure de thionyle doit être additionné goutte à goutte tout en maintenant le mélange méthanolique d'acides aminés à une température proche de 25°C (refroidissement par double enveloppe). L'agitation mécanique reste constante durant l'addition. Lorsque la totalité du SOCI2 a été additionnée, les acides aminés insolubles dans le méthanol se solubilisent totalement. Le mélange réactionnel est ensuite chauffé à reflux pendant deux heures. Le mélange obtenu est alors limpide de couleur marron foncé. Le méthanol et le SO2 en excès sont éliminés par distillation sous pression atmosphérique + pression d'azote puis en final sous vide. Les esters d'acides aminés sont repris à nouveau par 10 litres d'heptane puis évaporés sous vide à sec. Le pourcentage de Méthanol résiduel doit être inférieur à 1%. Répéter l'opération d'épuisement à l'heptane si nécessaire. Charger ensuite 70 litres de CH2CI2 et homogénéiser le mélange.

### 3/ Acylation des esters méthyliques d'acides aminés et du chlorure d'octanoyle

Le mélange réactionnel maintenu à 25°C est additionné de 14,1 Kg de triéthylamine (2,5 eq/AA esters soit 139,5 moles) puis à nouveau de 5 litres de dichlorométhane (rinçage ampoule). L'addition de TEA est réalisée à la température de 25°C, en fin de coulée la température est d'environ 30°C. Le mélange est ensuite agité énergiquement à cette température pendant deux heures afin de bien dissoudre l'ensemble des esters méthyliques (formation d'agrégats possible). Le mélange réactionnel obtenu est laiteux beige clair. Une fois homogénéisé, le mélange est acylé.

Pour l'opération d'acylation, 8,16 Kg de chlorure d'octanoyle (soit 0,90 eq/AA esters soit 50,15 moles) sont montés dans l'ampoule de coulée puis additionnés de 7,5 litres de CH2CI2. Le mélange acylant est ensuite additionné goutte à goutte sous agitation constante au mélange d'esters d'acides aminés. Durant l'addition, la température du mélange évolue exothermiquement de 25 à 35°C (laisser filer la température tout en contrôlant l'exotherme). Une fumée blanche caractéristique d'un dégagement de HCI apparaît. Une fois l'addition terminée, le mélange est agité pendant 1 heure puis chauffé à reflux pendant 1 heure.

### 4/ Lavage du mélange réactionnel (4 à 5 lavages)

La triéthylamine et les autres impuretés en excès présentes dans le mélange réactionnel sont éliminées grâce à plusieurs lavages à l'eau déminéralisée et un lavage à l'eau acidulée. Au total 4 à 5 opérations de lavage sont nécessaires.

### 1er lavage à l'eau déminéralisée :

Le mélange réactionnel est lavé par 20 litres d'eau déminéralisée qui sont additionnés directement dans le réacteur puis agité pendant 15 minutes. Une fois l'agitation stoppée, les phases sont transférées dans un décanteur. La phase organique (phase inférieure) environ 120-130 litres est soutirée et transférée dans le réacteur par pompage. La phase aqueuse (environ 35 litres) qui présente un pH basique (8-9) est éliminée.

### 2e lavage à l'eau acidifiée avec HCI concentré 37% :

Le mélange réactionnel est lavé par 20 litres d'eau déminéralisée acidifiée par 2,5 Kg de HCI 37%. Le mélange est agité pendant 30 minutes. Une fois l'agitation stoppée, les phases sont transférées dans un décanteur. La phase organique (phase inférieure) environ 120 litres est soutirée et transférée dans le réacteur par pompage. La phase aqueuse qui présente un pH acide (pH proche de 3) est éliminée.

### 3e lavage à l'eau déminéralisée (opération de lavage permettant de remonter le pH) :

Le mélange réactionnel est lavé par 20 litres d'eau déminéralisée qui sont additionnés directement dans le réacteur puis agité pendant 15 minutes. Une fois l'agitation stoppée, les phases sont transférées dans un décanteur. La phase organique (phase inférieure) environ 120-130 litres est soutirée et transférée dans le réacteur par pompage. La phase aqueuse qui présente un pH acide (pH proche de 4) est éliminée.

### 4e lavage à l'eau déminéralisée (opération de lavage permettant de remonter le pH) :

Le mélange réactionnel est lavé par 20 litres d'eau déminéralisée qui sont additionnés directement dans le réacteur puis agité pendant 15 minutes. Une fois l'agitation stoppée, les phases sont transférées dans un décanteur. La phase organique (phase inférieure) environ 120-130 litres est soutirée et transférée dans le réacteur par pompage. La phase aqueuse qui présente un pH acide (pH proche de 4 - 5) est éliminée.

### 5/ Séchage et concentration par évaporation de la phase chlorométhylénique

Les 120 litres de chlorure de méthylène sont filtrés puis remontés dans le réacteur et évaporés par distillation à Pression atmosphérique (en finalité sous vide). En fin de distillation, une huile jaune clair apparaît dans le réacteur. Additionner 50 litres d'éthanol et distiller environ 10 litres à pression atmosphérique pour épuiser le reste de CH2CI2. Les 40 litres de solution éthanolique renfermant les Thiolipesters de soie C8SMET 8515 doivent être limpide de couleur jaune paille.

### 6/ Traitement de la phase éthanolique au noir de carbone et au celatom

Le mélange éthanolique est ensuite additionné de 0,5 à 1 Kg de noir de carbone et 0,5 à 1 Kg de silice diatomée puis porté au reflux pendant 1h. Le mélange est ensuite filtré à chaud sur filtre à plaque. Un tourne en rond est réalisé pendant 30 minutes jusqu'à obtenir une solution éthanolique limpide et de couleur jaune très clair.

### 7/ Condensation du Thiolipesters de soie par évaporation de l'éthanol

La solution éthanolique renfermant les Thiolipesters de soie est ensuite transférée dans un second réacteur pour y être condensée. La distillation de l'éthanol est réalisée sous pression atmosphérique dans un premier temps puis sous vide en fin de concentration. Une huile de couleur jaune clair et odorante est obtenue.

### Exemple 2 -

### Test sur la viabilité cellulaire de kératinocytes humains normaux

Il s'agit de déterminer les concentrations non cytotoxiques du complexe lipidique selon l'invention - mis en présence de Kératinocytes Humains Normaux pendant 48H d'application. La méthode utilisée est celle du test cellulaire XTT.

Le système XTT est une méthodologie permettant de quantifier la viabilité de cellules par l'intermédiaire de leur activité mitochondriale déshydrogénase (méthode colorimétrique et non radioactive). Ce test est simple, précis et permet l'obtention de résultats reproductibles.

Ce test est basé sur le clivage du sel de tétrazolium XTT jaune en un dérivé formazan orange par le système " succinate-tétrazolium reductase " présent dans la chaîne respiratoire mitochondriale des cellules. Ainsi, cette conversion apparaît seulement au sein des cellules métaboliquement actives donc vivantes.

Le dérivé formazan est mesurable par spectrophotométrie à 450 nm.

Le kit "Cell Proliferation Kit II" (Roche) contient les réactif XTT et PMS (évite la bioréduction du XTT). Cette solution est préparée à 0,25 mg/ml dans du milieu de culture KSFM sans complément (Gibco).

### 1) Produits à tester

Le complexe lipidique ci-après dénommé complexe lipidique A comprend de l'huile de macadamia, de l'huile de cameline, de l'huile de pépins de raisin, au moins un ester de palmitate et au moins un ester de myristate et de la vitamine E.

Le complexe lipidique ci-après dénommé complexe lipidique B comprend tous les éléments du complexe lipidique A et au moins un N-acylamino ester enrichi en acides aminés soufrés.
* Complexe lipidique B selon l'invention dont Thio-lipesters 0,1% à 10% (m/m) dans l'éthanol absolu. Solution liquide contenue dans un flacon en verre. Chauffage à 50/55°C afin d'obtenir une limpidité correcte.
   Six Concentrations évaluées : 0,1% ; 0,05% ; 0,025% ; 0,0125% ; 0,006% et 0,003% (v/v) pour un temps d'incubation de 48H avec les cellules, dans du milieu KSFM.
* Complexe lipidique B selon l'invention dont Thio-lipesters 0,2%. Solution liquide contenue dans un flacon en verre. Réalisation d'une solution 10% (m/m) dans l'éthanol absolu.
   Quatre Concentrations évaluées : 0,1% ; 0,05% ; 0,025% et 0,0125% (v/v) pour un temps d'incubation de 48H avec les cellules, dans du milieu KSFM. Ce produit a été évalué dans une expérience indépendante des produits.
* Ethanol absolu

Quatre concentrations évaluées : 1/100 (1%) - 1/200 (0,5%) - 1/400 (0,25%) et 1/800 (0,125%) (v/v) pour un temps d'incubation de 48H avec les cellules, dans du milieu KSFM.

Ces concentrations correspondent aux 4 premières dilutions en éthanol respectives de chaque concentration de produit évalué car les produits sont concentrés dans l'éthanol absolu.

### 2) Préparation des cellules

Les kératinocytes humains normaux (KHN - primocultures) sont ensemencés à la densité de 10.000 cellules/puits (milieu de culture KSFM - Life Technologies) dans une microplaque 96 puits. La plaque est placée dans une étuve humide (37°C et 5% CO2) jusqu'à obtention de cellules confluentes à 80%.

### 3) Traitement des cellules

Le produit, testé à différentes concentrations dans le milieu de culture, est mis en présence des cellules pendant 48 H.

Chaque concentration de produit est évaluée en sextuple. Des cellules sont laissées sans traitement afin d'obtenir une condition témoin.

### 4) Mesure de la cytotoxicité

Après 48H de traitement, les puits de la microplaque sont rincés avec du PBS sans Ca++ ni Mg++ (Life Technologies) puis les cellules sont mises en présence de la solution XTT 0,25 mg/ml. La plaque est replacée à l'étuve humide (37°C, 5%CO2) dans l'obscurité. La solution XTT est également déposée dans des puits sans cellules afin de réaliser un blanc.

Après 4H, l'absorbance à 450 nm est mesurée.

### 5) Expression des résultats

Les mesures de densité optique (absorbance) des 6 puits traités avec la même concentration de produit sont moyennées. Cette moyenne est comparée à la moyenne des mesures obtenues pour les 6 puits témoin (test t de Student - comparaison des moyennes - différence significative à 95% si p<0,05* et à 99% si p<0,01**).

Les viabilités des cellules traitées sont exprimées en pourcentage par rapport au témoin (cellules non traitées) de 100% (DO traité / DO témoin x 100).

### 6) Résultats

Viabilité cellulaire en présence des produits

**Complexe lipidique dont Thio-lipesters 0,1% à 10% (m/m) dans l'éthanol absolu**

| | Témoin | 0,1% | 0,05% | 0,025% | 0,0125% | 0,006% | 0,003% |
|---|---|---|---|---|---|---|---|
| Absorbance moyenne 450 nm | 0,6991 ± 0,0666 | 0,6664 ± 0,0621 | 0,9026 ± 0,2645 | 0,9322 ± 0,3003 | 0,8541 ± 0,2725 | 0,8085 ± 0,2459 | 0,7884 ± 0,3103 |
| % viabilité | 100 | 95 | 129 | 133 | 122 | 116 | 113 |
| p (Student) | | 0,1996 | 0,0603 | 0,0587 | 0,1141 | 0,1677 | 0,2595 |

**Ethanol absolu**

| | Témoin | 1/100 | 1/200 | 1/400 | 1/800 |
|---|---|---|---|---|---|
| Absorbance moyenne 450 nm | 0,7589 ± 0,0559 | 0,4597 ± 0,1171 | 0,6632 ± 0,0817 | 0,6028 ± 0,0171 | 0,6205 ± 0,2414 |
| % viabilité | 100 | 61** | 87* | 79** | 82 |
| p (Student) | | 0,0003 | 0,0241 | 0,0002 | 0,1126 |

| | | | | | |
|---|---|---|---|---|---|
| ** : résultats significativement différents du témoin, à 99%. | | | | | |

**Complexe lipidique dont Thio-lipesters 0,2% à 10% (m/m) dans l'éthanol absolu**

| | Témoin | 0,1% | 0,05% | 0,025% | 0,0125% |
|---|---|---|---|---|---|
| Absorbance moyenne 450 nm | 0,4596 ± 0,0082 | 0,4680 ± 0,0068 | 0,4807 ± 0,0151 | 0,4771 ± 0,0159 | 0,4786 ± 0,0166 |
| % viabilité | 100 | 102 | 105 | 104 | 104 |
| p (Student) | | 0,0411 | 0,0066 | 0,0189 | 0,0152 |

Le produit complexe lipidique B dont Thio-lipesters 0,1 % n'affecte pas la viabilité des kératinocytes humains aux concentrations testées de 0,1% à 0,003 %.

Le produit complexe lipidique B dont Thio-lipesters 0,2 % n'est pas cytotoxique aux concentrations testées de 0,1 % à 0,0125%.

Nous constatons également une augmentation des valeurs de DO pour les produits complexe lipidique B dont Thio-lipesters 0,1 % à partir de la concentration 0,05 %. Ces résultats peuvent être en faveur d'une augmentation de la prolifération cellulaire.

### Exemple 3 -

### Test sur la prolifération de cellules humaines issues de la papille dermique de cheveu.

Le cheveu est constitué d'un follicule pileux ancré à 4mm de profondeur dans le derme. La papille dermique est un bourgeonnement d'origine dermique qui se trouve à la base du follicule constituant ainsi son point d'ancrage. Elle se compose de cellules conjonctives « fibroblaste-like » d'origine mésenchymale qui secrètent une importante matrice extracellulaire. Véritable réservoir de facteurs de croissance, la papille peut être considérée comme le coeur du follicule.

Les cellules HFDPC - Human Follicle Dermal Papilla Cells, sont issues de la papille dermique. Elles sont obtenues à partir du derme d'un scalp latéral et sont caractérisées par un marquage positif à l'alcaline phosphatase.

Il s'agit de déterminer si les produits complexe lipidique A et thio-lipesters induisent une prolifération cellulaire des cellules HFDPC au moyen d'un test d'incorporation de BrdU (pyrimidine 5-bromo-2'-deoxyuridine).

Il s'agit d'évaluer la prolifération de cellules HFDPC après traitement pendant 36H avec 4 produits.

Le test « Cell Proliferation ELISA, BrdU » permet de quantifier la prolifération cellulaire, par mesure de l'incorporation de BrdU pendant l'étape de synthèse d'ADN suivie d'une détection en luminescence. C'est une méthode sensible et non radioactive (alternative à l'incorporation de [H3] thymidine).

Les cellules sont mises en présence du traitement pour un temps défini (étuve 37°C, 5%CO2). Ensuite l'analogue pyrimidine 5-bromo-2'-deoxyuridine (BrdU) est ajouté aux cellules et celles-ci sont remises en incubation (étuve 37°C, 5%CO2) pour un temps de 12H. Pendant cette période le BrdU est incorporé à l'ADN des cellules proliférantes à la place de la thymidine.

Le milieu de culture est retiré, puis les cellules sont fixées et l'ADN dénaturé dans le même temps (accessibilité de l'anticorps).

Un anticorps anti BrdU-POD (peroxydase) est ajouté. Celui-ci se fixe au BrdU incorporé dans l'ADN nouvellement synthétisé.

Le complexe immunologique est alors détecté par ajout du substrat luminol puis lecture en luminescence (Genios, Tecan).

### 1) Produits testés

* Produit C : 10% complexe lipidique B dont Thio-lipester de soie 0,1% dans 90% d'éthanol absolu. Solution liquide contenue dans un flacon en verre. Chauffage à 50/55°C afin d'obtenir une limpidité correcte.
   Trois Concentrations évaluées : 0,1% ; 0,05% et 0,025% dans du milieu de culture basal FDPC (Promocell).
* Produit D : 10 % complexe lipidique B dont Thio-lipester de soie 0,2% dans 90% d'éthanol absolu. Solution liquide contenue dans un flacon en verre. Chauffage à 50/55°C afin d'obtenir une limpidité correcte.
   Trois Concentrations évaluées : 0,1% ; 0,05% et 0,025% dans du milieu de culture basal FDPC (Promocell).
* 90% Ethanol absolu (Prolabo 20820.327 lot 09G070506)

Trois concentrations évaluées : 1/100 (0,9%) - 1/200 (0,45%) - 1/400 (0,225%)

Ces concentrations sont définies par les 3 premières concentrations des produits car les produits sont concentrés dans l'éthanol absolu (10% de produit dans 90% d'éthanol).

EGF : Epidermal Growth Factor human, Sigma E-99644. Test à 10ng/ml.

### 2) Préparation des cellules

Les cellules HFDPC sont fournies par la société Promocell (Heidelberg, Germany). Elles ont été isolées à partir d'un donneur féminin, type caucasien, depuis le derme d'un scalp latéral (cheveux brun). Les cellules ont été réceptionnées à confluence dans du milieu de croissance FDPC Growth Medium.

Elles sont transférées en plaque 96 puits. La plaque est placée dans une étuve humide (37°C et 5% CO2) pendant 24H.

### 3) Traitement des cellules

Les produits testés à trois concentrations dans le milieu de culture, sont mis en présence des cellules pendant 36 H.

Chaque concentration de produit est évaluée en sextuple. Des cellules sont laissées sans traitement afin d'obtenir une condition témoin. Des cellules sont traitées avec de l'EGF afin d'obtenir une condition contrôle positif de prolifération.

### 4) Mesure de la prolifération

La quantification de la prolifération cellulaire s'effectue selon la méthode décrite dans le kit « Cell Proliferation ELISA, BrdU » (Roche Applied Science n°11669915001). Il s'agit d'un dosage de type immunologique, révélation en luminescence (Génios, Tecan).

### 5) Expression des résultats

Les mesures de luminescence (RLU/s) des 6 puits traités avec la même condition sont moyennées. Cette moyenne est comparée à la moyenne des mesures obtenues pour les 6 puits témoin (test t de Student - comparaison des moyennes - différence significative à 95% si p<0,05* et à 99% si p<0,01**).

L'augmentation de prolifération des cellules traitées en présence des produits est exprimé en pourcentage par rapport au témoin éthanol (dilution correspondante) de 100% [(RLU traité/RLU témoin)-1] x 100.

Pour le produit contrôle positif EGF, le pourcentage est exprimé par rapport au témoin non traité.

### 6) Résultats

EtOH : 90% Ethanol absolu (Prolabo 20820.327 lot 09G070506)

En présence des produits C et D, les pourcentages de prolifération sont exprimés versus l'éthanol correspondant à la concentration des produits. En présence d'EGF, le pourcentage de prolifération est exprimé versus le témoin.

**Concentration 0,1% en produit**

| | Témoin | EtOH 1/100 | C 0,1% | D 0,1% | EGF 10ng/ml |
|---|---|---|---|---|---|
| RLU | 420 ± 136 | 305 ± 108 | 554 ± 138 | 516 ± 73 | 1182 ± 109 |
| % Prolifération | | | 23% | 23% | 181% |
| p (Student) | | | ** | ** | ** |

| | | | | | |
|---|---|---|---|---|---|
| Différence significative à 99% si p<0,01** - ns : non significatif | | | | | |

**Concentration 0,05% en produit**

| | Témoin | EtOH 1/200 | C 0,05% | D 0,05% | EGF 10ng/ml |
|---|---|---|---|---|---|
| RLU | 282 ± 108 | 304 ± 89 | 522 ± 66 | 554 ± 117 | 1313 ± 184 |
| % Prolifération | | | 72% | 82% | 365% |
| p (Student) | | | ** | ** | ** |

| | | | | | |
|---|---|---|---|---|---|
| Différence significative à 99% si p<0,01** | | | | | |

**Concentration 0,025% en produit**

| | Témoin | EtOH 1/400 | C 0,025% | D 0,025% | EGF 10ng/ml |
|---|---|---|---|---|---|
| RLU | 405 ± 117 | 368 ± 70 | 412 ± 76 | 400 ± 62 | 726 ± 134 |
| % Prolifération | | | 12% | 9% | 80% |
| p (Student) | | | ns | ns | ** |

| | | | | | |
|---|---|---|---|---|---|
| Différence significative à 99% si p<0,01** - ns : non significatif | | | | | |

Les produits C - complexe lipidique A et thiolipesters de soie 0,1 %- et D -complexe lipidique A et thiolipesters de soie 0,2 % - ont été testés aux concentrations de 0,1% ; 0,05% et 0,025% sur la prolifération de cellules HFDPC (Human Follicular Dermal Papilla cells), isolées à partir du derme papillaire provenant de follicule du cuir chevelu humain.

Les produits C et D induisent une augmentation significative (p <0,01) de la prolifération cellulaire aux concentrations de 0,1% et 0,05 %.

Le tableau ci-après résume les résultats obtenus en % d'inhibition :

| | 0,1% | 0,05% | 0,025% |
|---|---|---|---|
| Complexe lipidique A et Thiolipesters de soie 0,1% | **82% | **72% | ns |
| Complexe lipidique A et Thiolipesters de soie 0,2 % | **70% | **82% | ns |

| | | | |
|---|---|---|---|
| ** p < 0,01 Ns : non significatif | | | |

Il n'y a pas de différence d'inhibition entre Complexe lipidique B dont thiolipesters de soie 0,1 % ou 0,2 %.

Pour la préparation des compositions cosmétiques ou dermatologiques, le complexe lipidique selon l'invention est mélangé aux excipients et aux additifs communément employés par l'homme de métier. La forme de la préparation peut être liquide, pâteuse ou solide. Il est également possible d'incorporer le complexe lipidique dans des vecteurs tels que les liposomes, les nanoparticules ou tout autre support permettant de véhiculer le complexe lipidique.

Des préparations renfermant les complexes lipidiques A ou B décrits dans l'invention et destinées à favoriser la croissance et le renouvellement des phanères sont données ci-après à titre d'exemple.
• Lotion fortifiante pour capillaires

| **INGREDIENTS** | **% (w/w)** |
|---|---|
| ALCOOL ÉTHYLIQUE | 28,8 |
| ISODODÉCANE | 6,4 |
| **COMPLEXE LIPIDIQUE B** | 1,0 |
| PARFUM (FRAGRANCE) | 0,5 |
| ACIDE CITRIQUE | 0,025 |
| AQUA (Purified water) | qsp 100 (balance) |

• Masque capillaire régénérant

| **INGREDIENTS** | **% (w/w)** |
|---|---|
| COCAMIDE DEA | 16,0 |
| **COMPLEXE LIPIDIQUE A** | 10,0 |
| BEURRE DE KARITÉ | 3,0 |
| D-PANTHENOL | 1,0 |
| PARFUM (FRAGRANCE) | 0,5 |
| ACIDE CITRIQUE | 0,025 |
| AQUA (Purified water) | qsp 100 (balance) |

• Ampoules fortifiantes pour capillaires

| **INGREDIENTS** | **% (w/w)** |
|---|---|
| ISODODÉCANE | 59,3 |
| **COMPLEXE LIPIDIQUE B** | 40,0 |
| PARFUM (FRAGRANCE) | 0,7 |

• Shampooing régénérant pour capillaires

| **INGREDIENTS** | **% (w/w)** |
|---|---|
| SODIUM COCETH SULFATE | 10,0 |
| COCOAMIDOPROPYL BETAÏNE | 7,0 |
| PEG 40 GLYCERYL COCOATE | 4,0 |
| COCAMIDE DEA | 4,0 |
| **COMPLEXE LIPIDIQUE A** | 3,0 |
| FRAGRANCE | Quantité nécessaire |
| CONSERVATEURS (Antiseptics) | Quantité nécessaire |
| AQUA (PURIFIED WATER) | qsp 100 (balance) |

• Shampooing fortifiant pour capillaires

| **INGREDIENTS** | **% (w/w)** |
|---|---|
| SODIUM COCETH SULFATE | 10,0 |
| SODIUM LAURYL SULFATE | 15,0 |
| COCOAMIDOPROPYL BETAÏNE | 7,0 |
| PEG 40 GLYCERYL COCOATE | 4,0 |
| COCAMIDE DEA | 3,0 |
| **COMPLEXE LIPIDIQUE B** | 3,0 |
| FRAGRANCE | Quantité nécessaire |
| CONSERVATEURS (Antiseptics) | Quantité nécessaire |
| AQUA (PURIFIED WATER) | qsp 100 (balance) |

• Composition lotion cheveux gras

| **INGREDIENTS** | **% (w/w)** |
|---|---|
| ALCOOL DÉNATURÉ | 45,0 |
| **COMPLEXE LIPIDIQUE B** | 15,0 |
| ROSMARINUS OFFICINALIS | 0,2 |
| THYMUS VULGARIS | 1,0 |
| AQUA (PURIFIED WATER) | qsp 100 (balance) |

## Revendications

1. Complexe lipidique pour son utilisation thérapeutique pour favoriser la croissance des phanères
**caractérisé par le fait qu'**il comprend un mélange d'huile de macadamia représentant de 30 à 55 % en poids du complexe, d'huile de cameline représentant de 20 à 35 % en poids du complexe et d'huile de pépins de raisin représentant de 9 à 11 % en poids du complexe, au moins un ester d'acide myristique d'origine végétale représentant de 1 à 15% en poids du complexe et au moins un ester d'acide palmitique d'origine végétale représentant de 5 à 30 % en poids du complexe.

2. Complexe selon la revendication précédente dans lequel les esters sont choisis parmi les esters méthyliques, éthyliques, propyliques ou isopropyliques.

3. Complexe selon l'une quelconque des revendications précédentes comprenant au moins un N-acylamino ester obtenu par transformation chimique d'hydrolysat de protéines animales ou végétales préalablement enrichi en acides aminés soufrés.

4. Complexe selon la revendication précédente dans lequel le au moins un N-acylamino ester est synthétisé à partir d'hydrolysat de soie.

5. Complexe selon l'une quelconque des deux revendications précédentes dans lequel au moins un acide aminé soufré est choisi parmi la cystine, la cystéine, l'homocystéine, la méthionine et la taurine pris individuellement ou en mélange.

6. Complexe selon l'une quelconque des trois revendications précédentes dans lequel le au moins un N-acylamino ester enrichi en acides aminés soufrés représente de 0,1 à 1 % en poids du complexe.

7. Complexe selon l'une quelconque des revendications précédentes comprenant de la vitamine E.

8. Complexe selon l'une quelconque des revendications précédentes comprenant en poids du poids total du complexe :
| | |
|---|---|
| - Huile de macadamia | 44,0 % (± 20 %) ; |
| - Huile de cameline | 26,0 % (± 20 %) ; |
| - Huile de pépins de raisins | 10,0 % (± 10 %) ; |
| - Palmitate d'isopropyle | 17,0 % (± 10 %) ; |
| - Myristate d'isopropyle | 3,0 % (± 10 %) ; |
| - Vitamine E | 0,2 % (± 5 %). |

9. Complexe selon l'une quelconque des revendications précédentes comprenant en poids du poids total du complexe :
| | |
|---|---|
| - Huile de macadamia | 44,0 % ; |
| - Huile de cameline | 26,0 % ; |
| - Huile de pépins de raisins | 10,0 % ; |
| - Palmitate d'isopropyle | 17,0 % ; |
| - Myristate d'isopropyle | 2,7 % ; |
| - Vitamine E | 0,2 % ; |
| - N-acylamino ester de soie enrichi en acides aminés soufrés | 0,1 %. |

10. Utilisation cosmétique et non thérapeutique d'un complexe lipidique comprenant un mélange d'huile de macadamia représentant de 30 à 55 % en poids du complexe, d'huile de cameline représentant de 20 à 35 % en poids du complexe et d'huile de pépins de raisin représentant de 9 à 11 % en poids du complexe, au moins un ester d'acide myristique d'origine végétale représentant de 1 à 15 % en poids du complexe et au moins un ester d'acide palmitique d'origine végétale représentant de 5 à 30 % en poids du complexe pour favoriser la croissance de phanères.

11. Utilisation cosmétique et non thérapeutique selon la revendication précédente dans laquelle les esters sont choisis parmi les esters méthyliques, éthyliques, propyliques ou isopropyliques.

12. Utilisation cosmétique et non thérapeutique selon l'une quelconque des deux revendications précédentes comprenant au moins un N-acylamino ester obtenu par transformation chimique d'hydrolysat de protéines animales ou végétales préalablement enrichi en acides aminés soufrés.

13. Utilisation cosmétique et non thérapeutique selon la revendication précédente dans laquelle le au moins un N-acylamino ester est synthétisé à partir d'hydrolysat de soie.

14. Utilisation cosmétique et non thérapeutique selon l'une quelconque des deux revendications précédentes dans laquelle au moins un acide aminé soufré est choisi parmi la cystine, la cystéine, l'homocystéine, la méthionine et la taurine pris individuellement ou en mélange.

15. Utilisation cosmétique et non thérapeutique selon l'une quelconque des trois revendications précédentes dans laquelle le au moins un N-acylamino ester enrichi en acides aminés soufrés représente de 0,1 à 1 % en poids du complexe.

16. Utilisation cosmétique et non thérapeutique selon l'une quelconque des six revendications précédentes comprenant de la vitamine E.

17. Utilisation cosmétique et non thérapeutique selon l'une quelconque des sept revendications précédentes comprenant en poids du poids total du complexe :
| | |
|---|---|
| - Huile de macadamia | 44,0 % (± 20 %) ; |
| - Huile de cameline | 26,0 % (± 20 %) ; |
| - Huile de pépins de raisins | 10,0 % (± 10 %) ; |
| - Palmitate d'isopropyle | 17,0 % (± 10 %) ; |
| - Myristate d'isopropyle | 3,0 % (± 10 %) ; |
| - Vitamine E | 0,2 % (± 5 %). |

18. Utilisation cosmétique et non thérapeutique selon l'une quelconque des huit revendications précédentes comprenant en poids du poids total du complexe :
| | |
|---|---|
| - Huile de macadamia | 44,0 % ; |
| - Huile de cameline | 26,0 % ; |
| - Huile de pépins de raisins | 10,0 % ; |
| - Palmitate d'isopropyle | 17,0 % ; |
| - Myristate d'isopropyle | 2,7 % ; |
| - Vitamine E | 0,2 % ; |
| - N-acylamino ester de soie enrichi en acides aminés soufrés | 0,1 %. |

19. Utilisation cosmétique et non thérapeutique pour favoriser la croissance des phanères d' une composition cosmétique où le complexe lipidique selon l'une des revendications 1 à 9 représente 1 à 50% en poids de la composition.

20. Utilisation cosmétique et non thérapeutique selon la revendication précédente la composition cosmétique étant sous forme de lotion capillaire, de masque capillaire, d'ampoule capillaire ou de shampoing capillaire.

21. Utilisation cosmétique et non thérapeutique selon la revendication 19 la composition cosmétique étant sous forme de liquide ou crème pour les ongles ou les cils.

22. Composition dermatologique destinée à une utilisation thérapeutique pour favoriser la croissance des phanères comprenant le complexe lipidique selon l'une quelconque des revendications 1 à 9.

23. Composition pour son utilisation selon la revendication précédente dans laquelle le complexe lipidique représente de 1 à 50% en poids de la composition.

24. Composition pour son utilisation selon l'une quelconque des deux revendications précédentes sous forme de lotion capillaire, de masque capillaire, d'ampoule capillaire ou de shampoing capillaire.

25. Composition pour son utilisation selon l'une quelconque des revendications 22 et 23 sous forme de liquide ou crème pour les ongles ou les cils.

## Patentansprüche

1. Lipidkomplex zu dessen therapeutischer Anwendung zur Förderung des Wachstums von Hautanhangsgebilden, **dadurch gekennzeichnet, dass** er eine Mischung aus Macadamiaöl zu 30 bis 55 % Gewichtsanteil des Komplexes, Leindotteröl zu 20 bis 35 % des Gewichtsanteils das Komplexes und Traubenkernöl zu 9 bis 11 % Gewichtsanteil des Komplexes umfasst, mindestens ein Myristinsäureester pflanzlichen Ursprungs, das 1 bis 15 % Gewichtsanteil des Komplexes ausmacht und mindestens ein Palmitinsäureester pflanzlichen Ursprung, dass 5 bis 30 % Gewichtsanteil des Komplexes ausmacht.

2. Komplex nach dem vorhergehenden Anspruch, in dem die Ester aus Methylester, Ethylester, Propylester oder Isopropylester ausgewählt werden.

3. Komplex nach einem der vorhergehenden Ansprüche, welcher mindestens ein N-Acylaminosäureester umfasst, dass durch die chemische Umwandlung eines Hydrolysats aus tierischem oder pflanzlichem Eiweiß, das zuvor mit schwefelhaltigen Aminosäuren angereichert wurde, erzielt wird.

4. Komplex nach dem vorhergehenden Anspruch, in dem der oder mindestens ein N-Acylaminosäureester aus Seidenhydrolysat synthetisiert wird.

5. Komplex nach einem der beiden vorhergehenden Ansprüche, in dem mindestens eine schwefelhaltige Aminosäure aus Cystin, Cystein, Homocystein, Methionin und Taurin jeweils alleine oder gemischt ausgewählt wird.

6. Komplex nach einem der drei vorhergehenden Ansprüche, indem das oder mindestens ein mit schwefelhaltigen Aminosäuren angereichertes N-Acylaminosäureester einen Gewichtsanteil das Komplexes von 0,1 bis 1 Prozent ausmacht.

7. Komplex nach einem der vorhergehenden Ansprüche, der Vitamin E umfasst.

8. Komplex nach einem der vorhergehenden Ansprüche, der folgenden Gewichtsanteile des Gesamtgewichtes des Komplexes umfasst:
| | |
|---|---|
| - Macadamiaöl | 44,0 % (± 20 %); |
| - Leindotteröl | 26 % (± 20 %); |
| - Traubenkernöl | 10,0 % (± 10 %); |
| - Isopropylpalmitat | 17,0 % (± 10 %); |
| - Isopropylmyristat | 3,0 % (± 10 %); |
| - Vitamin E | 0,2 % (± 5 %). |

9. Komplex nach einem der vorhergehenden Ansprüche, der folgende Gewichtsanteile am Gesamtgewicht des Komplexes umfasst:
| | |
|---|---|
| - Macadamiaöl | 44,0 %; |
| - Leindotteröl | 26 %; |
| - Traubenkernöl | 10,0 %; |
| - Isopropylpalmitat | 17,0 %; |
| - Isopropylmyristat | 2,7 %; |
| - Vitamin E | 0,2 % |
| - mit schwefelhaltigen Aminosäuren angereichertes N-Acylaminosäureester | 0,1 %. |

10. Kosmetische, nicht therapeutische Anwendung eines Lipidkomplexes zur Förderung des Wachstums von Hautanhangsgebilden, welcher eine Mischung aus Macadamiaöl mit 30 bis 55 % Gewichtsanteil des Komplexes, Leindotteröl mit 20 bis 35 % Gewichtsanteil des Komplexes und Traubenkernöl mit 9 bis 11 % Gewichtsanteil des Komplexes umfasst, mindestens ein Myristinsäureester pflanzlichen Ursprungs mit 1 bis 15 % Gewichtsanteil des Komplexes und mindestens ein Palmitinsäureester pflanzlichen Ursprungs mit 5 bis 30 % Gewichtsanteil.

11. Kosmetische, nicht therapeutische Anwendung nach dem vorhergehenden Anspruch, in welcher die Ester aus Methylester, Ethylester, Propylester oder Isopropylester ausgewählt werden.

12. Kosmetische, nicht therapeutische Anwendung nach einem der beiden vorhergehenden Ansprüche, welche mindestens ein N-Acylaminosäureester umfasst, dass durch die chemische Umwandlung von Hydrolysat aus tierischem oder pflanzlichen Eiweiß, das zuvor mit schwefelhaltigen Aminosäuren angereichert wurden, erzielt wird.

13. Kosmetische, nicht therapeutische Anwendung nach dem vorhergehenden Anspruch, in welcher der oder mindestens ein N-Acylaminosäureester aus Seidenhydrolysat synthetisiert wird.

14. Kosmetische, nicht therapeutische Anwendung nach einem der beiden vorhergehenden Ansprüche, in welcher mindestens eine schwefelhaltige Aminosäure aus Cystin, Cystein, Homocystein, Methionin und Taurin jeweils allein oder gemischt ausgewählt wird.

15. Kosmetische, nicht therapeutische Anwendung nach einem der drei vorhergehenden Ansprüche, in welcher der oder mindestens ein N-Acylaminosäureester, das mit schwefelhaltigen Aminosäuren angereichert wurde, 0,1 bis 1 % Gewichtsanteil des Komplexes ausmacht.

16. Kosmetische, nicht therapeutische Anwendung nach einem der sechs vorhergehenden Ansprüche, welche Vitamin E umfasst.

17. Kosmetische, nicht therapeutische Anwendung nach einem der sieben vorhergehenden Ansprüche, welche die folgenden Gewichtsanteile am Gesamtgewicht des Komplexes umfasst:
| | |
|---|---|
| - Macadamiaöl | 44,0 % (± 20 %); |
| - Leindotteröl | 26 % (± 20 %); |
| - Traubenkernöl | 10,0 % (± 10 %); |
| - Isopropylpalmitat | 17,0 % (± 10 %); |
| - Isopropylmyristat | 3,0 % (± 10 %); |
| - Vitamin E | 0,2 % (± 5 %). |

18. Kosmetische, nicht therapeutische Anwendung nach einem der acht vorhergehenden Ansprüche, welche folgende Gewichtsanteile am Gesamtgewicht des Komplexes umfasst:
| | |
|---|---|
| - Macadamiaöl | 44,0 %; |
| - Leindotteröl | 26 %; |
| - Traubenkernöl | 10,0 %; |
| - Isopropylpalmitat | 17,0 %; |
| - Isopropylmyristat | 2,7 %; |
| - Vitamin E | 0,2 % |
| - mit schwefelhaltigen Aminosäuren angereichertes N-Acylaminosäureester | 0,1 %. |

19. Kosmetische, nicht therapeutische Anwendung einer kosmetischen Zusammensetzung zur Förderung des Wachstums von Hautanhangsgebilden, in welcher der Lipidkomplex nach einem der Ansprüche 1 bis 9 1 bis 50 % Gewichtsanteil der Zusammensetzung ausmacht.

20. Kosmetische, nicht therapeutische Anwendung nach dem vorhergehenden Anspruch, in dem die kosmetische Zusammensetzung die Form einer Haarlotion, Haarmaske, Haarampulle oder eines Haarshampoos hat.

21. Kosmetische, nicht therapeutische Anwendung nach Anspruch 19, wobei die kosmetische Zusammensetzung die Form einer Flüssigkeit oder Creme für Nägel oder Wimpern hat.

22. Dermatologische Zusammensetzung, die zur therapeutischen Anwendung zur Förderung des Wachstums von Hautanhangsgebilden bestimmt ist, welche den Lipidkomplex nach einem der Ansprüche 1 bis 9 umfasst.

23. Zusammensetzung zur Anwendung nach dem vorhergehenden Anspruch, in welcher der Lipidkomplex 1 bis 50 % Gewichtsanteil der Zusammensetzung ausmacht.

24. Zusammensetzung zur Anwendung nach einem der beiden vorhergehenden Ansprüche in Form einer Haarlotion, Haarmaske, Haarampulle oder eines Haarshampoos.

25. Zusammensetzung zur Anwendung nach einem der vorhergehenden Ansprüche 22 und 23 in Form einer Flüssigkeit oder einer Creme für Nägel oder Wimpern.

## Claims

1. Lipid complex for its therapeutic use in promoting growth of the appendages, **characterised in that** it comprises a mixture of macadamia oil representing between 30 and 55% in weight of the complex, camelina oil representing between 20 and 35% in weight of the complex and grapeseed oil representing between 9 and 11% in weight of the complex, at least one plant-derived myristic acid ester representing between 1 and 15% in weight of the complex and at least one plant-derived palmitic acid ester representing between 5 and 30% in weight of the complex.

2. Complex according to the preceding claim, wherein the esters are selected from among methyl, ethyl, propyl or isopropyl esters.

3. Complex according to any one of the preceding claims, comprising at least one N-acylamino ester obtained by chemical transformation of animal or vegetable protein hydrolysate previously enriched with sulphur-containing amino acids.

4. Complex according to the preceding claim, wherein the at least one N-acylamino ester is synthesised from silk hydrolysate.

5. Complex according to any one of the two preceding claims wherein at least one sulphur-containing amino acid is selected from among cystine, cysteine, homocysteine, methionine and taurine considered individually or in a mixture.

6. Complex according to any one of the three preceding claims, wherein the at least one N-acylamino ester enriched with sulphur-containing amino acids represents between 0.1 and 1% in weight of the complex.

7. Complex according to any one of the preceding claims, containing vitamin E.

8. Complex according to any one of the preceding claims, containing in weight of the total weight of the complex:
| | |
|---|---|
| - macadamia oil | 44.0 % (± 20 %); |
| - camelina oil | 26.0 % (± 20 %); |
| - grapeseed oil | 10.0 % (± 10 %); |
| - isopropyl palmitate | 17.0 % (± 10 %); |
| - isopropyl myristate | 3.0 % (± 10 %); |
| - vitamin E | 0.2 % (± 5 %). |

9. Complex according to any one of the preceding claims, containing in weight of the total weight of the complex:
| | |
|---|---|
| - macadamia oil | 44.0%; |
| - camelina oil | 26.0%; |
| - grapeseed oil | 10.0 %; |
| - isopropyl palmitate | 17.0 %; |
| - isopropyl myristate | 2.7%; |
| - vitamin E | 0.2%; |
| - N-acylamino ester from silk enriched with sulphur-containing amino acids | 0.1 %. |

10. Cosmetic and non-therapeutic use of a lipid complex, comprising a mixture of macadamia oil representing between 30 and 55% in weight of the complex, camelina oil representing between 20 and 35% in weight of the complex and grapeseed oil representing between 9 and 11% in weight of the complex, at least one plant-derived myristic acid ester representing between 1 and 15 % in weight of the complex and at least one plant-derived palmitic acid ester representing between 5 and 30% in weight of the complex, in promoting growth of the appendages.

11. Cosmetic and non-therapeutic use according to the preceding claim wherein the esters are selected from among methyl, ethyl, propyl or isopropyl esters.

12. Cosmetic and non-therapeutic use according to any one of the two preceding claims, comprising at least one N-acylamino ester obtained by chemical transformation of animal or vegetable protein hydrolysate previously enriched with sulphur-containing amino acids.

13. Cosmetic and non-therapeutic use according to the preceding claim, wherein the at least one N-acylamino ester is synthesised from silk hydrolysate.

14. Cosmetic and non-therapeutic use according to any one of the two preceding claims wherein at least one sulphur-containing amino acid is selected from among cystine, cysteine, homocysteine, methionine and taurine considered individually or in a mixture.

15. Cosmetic and non-therapeutic use according to any one of the three preceding claims, wherein the at least one N-acylamino ester enriched with sulphur-containing amino acids represents between 0.1 and 1% in weight of the complex.

16. Cosmetic and non-therapeutic use according to any one of the six preceding claims, containing vitamin E.

17. Cosmetic and non-therapeutic use according to any one of the seven preceding claims, containing in weight of the total weight of the complex:
| | |
|---|---|
| - macadamia oil | 44.0 % (± 20 %); |
| - camelina oil | 26.0 % (± 20 %); |
| - grapeseed oil | 10.0 % (± 10 %); |
| - isopropyl palmitate | 17.0 % (± 10 %); |
| - isopropyl myristate | 3.0 % (± 10 %); |
| - vitamin E | 0.2 % (± 5 %). |

18. Cosmetic and non-therapeutic use according to any one of the eight preceding claims, containing in weight of the total weight of the complex:
| | |
|---|---|
| - macadamia oil | 44.0%; |
| - camelina oil | 26.0%; |
| - grapeseed oil | 10.0 %; |
| - isopropyl palmitate | 17.0 %; |
| - isopropyl myristate | 2.7%; |
| - vitamin E | 0.2%; |
| - N-acylamino ester from silk enriched with sulphur-containing amino acids | 0.1 %. |

19. Cosmetic and non-therapeutic use in promoting growth of the appendages of a cosmetic composition wherein the lipid complex according to any of claims 1 to 9 represents between 1 and 50% in weight of the composition.

20. Cosmetic and non-therapeutic use according to the preceding claim, wherein the cosmetic composition is in the form of a hair lotion, hair mask, hair ampoule or hair shampoo.

21. Cosmetic and non-therapeutic use according to the claim 19, wherein the cosmetic composition is in the form of a liquid or cream for the nails or eyelashes.

22. Dermatological composition intended for therapeutic use in promoting growth of the appendages, comprising the liquid complex according to any one of claims 1 to 9.

23. Composition for its use according to the preceding claim, wherein the lipid complex represents between 1 and 50% in weight of the composition.

24. Composition for its use according to any one of the two preceding claims in the form of a hair lotion, hair mask, hair ampoule or hair shampoo.

25. Composition for its use according to any one of claims 22 and 23 in the form of a liquid or cream for the nails or eyelashes.
